# EUROPEAN PATENT SPECIFICATION

(11) **EP 1 494 650 B1**
(45) Date of publication and mention of the grant of the patent: **04.05.2011**
(21) Application number: 03746393.2
(22) Date of filing: 20.03.2003
(51) Int. Cl.: A61K 31/4045, A61K 31/437, A61K 31/496, A61K 31/4985, A61K 45/06, A61K 9/20, A61P 25/20

(54) **PHARMACEUTICAL FORMULATION COMPRISING MELATONIN**
MELATONIN ENTHALTENDE PHARMAZEUTISCHE FORMULIERUNG
COMPOSITION PHARMACEUTIQUE CONTENANT DE LA MELATONINE

(30) Priority: 08.04.2002 IL 14937702
(43) Date of publication of application: 12.01.2005
(73) Proprietor: NEURIM PHARMACEUTICALS (1991) LIMITED, Tel Aviv 69710 (IL)
(72) Inventor: ZISAPEL, Nava, 69355 Tel Aviv (IL)
(74) Representative: Cawley, Aimee Elizabeth
(86) International application number: PCT/IL2003/000240
(87) International publication number: WO 2003/086352

(56) References cited:
- WO-A-97/32871
- WO-A-99/63977
- US-A- 6 034 105
- US-B1- 6 274 635
- SUHNER A ET AL: "EFFECTIVENESS AND TOLERABILITY OF MELATONIN AND ZOLPIDEM FOR THE ALLEVIATION OF JET LAG" AVIATION, SPACE AND ENVIRONMENTAL MEDICINE, AEROSPACE MEDICAL ASSOCIATION, ALEXANDRIA, VA, US, vol. 72, no. 7, July 2001 (2001-07), pages 638-646, XP008055456 ISSN: 0095-6562
- KIRKWOOD C K: "MANAGEMENT OF INSOMNIA" JOURNAL OF THE AMERICAN PHARMACEUTICAL ASSOCIATION, ASSOCIATION, WASHINGTON, DC, US, vol. 39, no. 5, September 1999 (1999-09), pages 688-696, XP008055724 ISSN: 1086-5802
- TEVOR R NORMAN AND OTHERS: "The effect of single oral doses of Zopiclone on nocturnal melatonin secretion in healthy male volunteers" PROGRESS IN NEURO-PSYCHOPHARMACOLOGY AND BIOLOGICAL PSYCHIATRY, vol. 25, 2001, pages 825-833, XP002382499

## Description

### FIELD OF THE INVENTION

The present invention relates to use of melatonin in the manufacture of medicaments for short-term potentiation of certain hypnotics, and to pharmaceutical formulations comprising melatonin and such hypnotics.

### BACKGROUND OF THE INVENTION

Gamma-aminobutyric acid (GABA) acting via GABA-A receptors is the brain's major inhibitory neurotransmitter system and exerts a crucial role in regulating brain excitability. GABA-A receptors comprise five subunits. The different protein subunits that make up the receptor for the inhibitory neurotransmitter gamma-aminobutyric acid (GABA) have been identified, and make up the alpha, beta, gamma and delta families, for each of which exist several subtypes. The subunit make-up of a receptor, particularly its alpha-subunit content, determines its pharmacological characteristics. A number of drugs interact with binding sites on different subunits of the GABA-A receptors, and these include modern hypnotic drugs (i.e. benzodiazepines, and the newer non-barbiturate and non-benzodiazepine agents, e.g. imidazopyridines and cyclopyrrolones), as well as anticonvulsants, anaesthetics and neurosteroids (e.g. the progesterone metabolite pregnalone).

Receptor subtype specificity of hypnotics has been explained in terms of differential affinity for receptors containing different alpha subunits, which are expressed in different brain regions. Thus, receptors that include an alpha1 subunit have a type (I) pharmacology and bind the non-barbiturate and non-benzodiazepine agents zolpidem and zaleplon with high affinity, whilst receptors with alpha2, alpha3 or alpha5 subunits have a type (II) pharmacology and bind these drugs with low affinity. Both type (I) and (II) bind diazepam and other benzodiazepines. In contrast, receptors that contain alpha4 and alpha6 subunits, are diazepam-insensitive. The ligand selectivity of receptor subunits assists in their characterization. Site-directed mutagenesis has indicated that benzodiazepines bind to a cleft on the GABA-A receptor surface at the interface between the alpha and gamma subunits. Other drugs (flumazenil, zopiclone, zolpidem) also bind to the alpha subunit, but interact with amino acids in different binding domains to the benzodiazepines.

Using immunochemical and ligand-binding techniques, the subunit composition of GABA-A receptors has been shown to exhibit a degree of brain regional specificity. The predominant GABA-A receptor composition found in the brain is alpha1beta2gamma2, which are all encoded on human chromosome 5. Targeted gene disruption has provided clues to the physiological functions served by GABA-A receptors containing different subunits. Receptors containing gamma2 appear to have a vital role in maintaining appropriate central inhibition, beta3-containing receptors may also be important determinants of excitability in certain brain regions, whereas a clear role for alpha5-, alpha6- and gamma3-containing receptors has not yet been established by these techniques.

GABA-A receptors are of great clinical significance in several disorders, including insomnia, epilepsy, anxiety and alcoholism; benzodiazepines are used commonly to treat anxiety, and studies suggest that benzodiazepine antagonists and inverse agonists (which induce the opposite effect to agonists at receptors) may be useful in alcohol rehabilitation.

Among the most prominent uses of GABA-A receptor modulators (benzodiazepines and non-benzodiazepine hypnotics) is the treatment of insomnia, defined as problems initiating and/or maintaining sleep, at least three nights/week accompanied by daytime distress or impairment. Persistent insomnia is associated with an array of individual and societal consequences, including greater medical and psychiatric morbidity, life-threatening accidents, reduced quality of life, impaired job performance, and absenteeism. Insomnia is associated with negative consequences for health-related quality of life, daytime well-being, and also has economic implications. The cost of insomnia in terms of lost productivity and accidents has been estimated at $77-$92 billion annually.

Benzodiazepines are very potent in sleep induction (shortening sleep latency) and maintenance (increasing total sleep time). These drugs have however detrimental effects on awakening from sleep (hangover effects) and daytime vigilance (psychomotor functioning), the next morning. The newer non-barbiturate and non-benzodiazepine hypnotic agents (e.g. imidazopyridines and cyclopyrrolones) have been available since the late 1980's and have been proposed as an alternative strategy. These shorten sleep latency and do not produce major "hangover" effects the next morning. The possible adverse effects of these sleep aids include residual sedation and psychomotor impairment, daytime anxiety, anterograde amnesia and cognitive impairment, rebound insomnia, and drug tolerance and dependence. Because patients may experience daytime sleepiness there is a potential for impaired performance and an increased risk of accidents, particularly of traffic accidents. All benzodiazepines adversely affect cognition by disrupting both short and long term memory. Episodic, semantic and iconic memory are impaired. Former use of benzodiazepines is associated with a significantly increased risk of dementia in elderly persons (65 years of age and older). The degree of memory loss is a function of the specific agent and dose. Therefore, lowering the dose of these agents, while maintaining their hypnotic effects, may be beneficial to circumvent these impairments.

The development of dependence on these drugs is also a matter of concern. The molecular mechanism of hypnotic dependence has been explored, and seems to involve down-regulation of transcription of the normally prevalent alpha1, beta2 and gamma2 subunits, and the reciprocal up-regulation of the expression of rarer subunits. Zolpidem is an imidazopyridine agent that is indicated for the short term (up to 4 weeks) treatment of insomnia, at a recommended dosage of 10 mg/day in adults and 5 or 10 mg/day in the elderly or patients with hepatic impairment. Chronic treatment with hypnotic drugs such as zopiclone and zolpidem, appears to produce more limited change in GABA-A receptor subunit expression. It has been shown that the hypnotic efficacy of zolpidem is generally comparable to that of the benzodiazepines flunitrazepam, flurazepam, nitrazepam, temazepam and triazolam as well as non-barbiturate and non-benzodiazepine hypnotic agents such as zopiclone and trazodone in the treatment of elderly and adult patients with insomnia.

Zaleplon is N-[3-(3-cyanopyrazolo[1,5-a]pyrimidin-7-yl)phenyl]-N-ethylacetamide; zolpidem is N,N,6-trimethyl-2-p-toyl-imidazo[1,2,-a]pyridine-3-acetamide L-(+)-tartrate (2:1); zopiclone is 6-(5-chloropyrid-2-yl)-5-(4-methylpiperazin-1-yl)carbonyloxy-7-oxo-6,7-dihydro-5H-pyrrolo[3,4-b]pyrazine; trazodone is 2-[3-{4-(*m*-chlorophenyl)-1-piperazinyl}propyl]s-triazolo[4,3-a]-pyridine-3(2*H*)-one monohydrochloride.

Zolpidem, for example, is gaining favour worldwide because of its efficacy and its side effect profile, which is milder and less problematic than that of the benzodiazepines and barbiturates used to treat insomnia. There is little evidence of rebound insomnia or withdrawal symptoms after discontinuation of the drug when it is given as recommended (10 mg/day for < 1 month) or over longer periods. Initially, there were no reports of tolerance developing to the hypnotic effects of zolpidem in a number of studies of up to 6 months duration. Still, side effects (delirium, hallucinations) are not uncommon with zolpidem use and it may have a marked dependence potential. Yet, in a recent report of a WHO Expert Committee responsible for reviewing information on dependence-producing drugs to assess the need for their international control, zolpidem was recommended for international control. Lowering the risk of developing dependence is thus a public health issue.

Daily cycles in physiology and behaviour appear to be a universal feature of living organisms. An intrinsic body clock residing in the brain's suprachiasmatic nucleus (SCN) regulates a complex series of rhythms including sleep-wakefulness. The individual period of the endogenous clock is either slower or faster than the solar 24-h day/night cycle (in humans it is usually >24 h) and is normally entrained by the 24-h light dark cycle to match the environmental rhythm. Light is the ubiquitous signal for resetting the timing of the clock. An important output signal generated by the SCN is the induction of synthesis of the pineal hormone melatonin (N-acetyl-5-methoxytryptamine) at night. Melatonin is directly regulated by the SCN and thus serves as a marker of the circadian clock phase; but it can also relay time-of-day information (signal of darkness) to various organs, including the SCN itself. The phase shifting effects of melatonin are essentially opposite to those of light. Thus, melatonin, given several hours before its endogenous peak at night, effectively advanced sleep time in delayed sleep phase syndrome patients and adjusted the sleep wake cycle to 24 h in the blind, where light therapy is inapplicable. Melatonin and light, when properly timed (namely light in the subjective night and melatonin in the subjective day of the internal clock) may also alleviate jet lag and sleep in night-shift workers trying to sleep during daytime.

Melatonin plays a major role in the induction and regulation of sleep. The sleep promoting activity of melatonin in humans is best demonstrated in daytime, when the hormone is not produced endogenously, or in subjects who suffer from abnormal melatonin production due to aging disease or use of certain drugs (e.g. beta adrenoceptor blockers). A number of pharmacodynamic interactions between melatonin and benzodiazepine-mediated behavioral effects have been reported. Benzodiazepine therapy has been found to suppress the nocturnal rise in plasma melatonin and shift its day-night rhythmicity; this suppression may interfere with normal sleep-wake rhythmicity and add-on melatonin replacement may help maintain the efficacy of benzodiazepine hypnotics. Thus, administration of sustained release melatonin (2 mg) to 23 chronic benzodiazepine-using elderly insomniacs, resulted in a significant improvement in sleep maintenance and total sleep time compared to placebo.

Besides replenishing the endogenous melatonin levels, melatonin was also reported to allow reduction of the therapeutic dose of the benzodiazepine triazolam by 50% while maintaining its hypnotic activity. These results could be ascribed to additive effects of melatonin and benzodiazepines of sleep induction. Most importantly, the sleep inducing, anxiolytic and anticonvulsant properties of melatonin are not mediated by the benzodiazepine receptor, since flumazenil, a benzodiazepine-antagonist, administered concomitantly was unable to block melatonin's effects.

Melatonin is also an effective aid in withdrawal for addictive drugs, including benzodiazepines. A strong proof of melatonin's efficacy in withdrawal from an addictive drug has been found when applied in nicotine withdrawal, which is usually accompanied by negative mood and performance. In addition, administration of melatonin enabled rapid discontinuation of benzodiazepine therapy in a 43-year-old woman who was benzodiazepine addicted. The effects of concomitant sustained release melatonin (2 mg/day), compared to placebo, in facilitating benzodiazepine discontinuation, was assessed in 34 adult volunteers (40-90 years old) with insomnia, who had been long term benzodiazepine users. The results indicated that sustained release melatonin effectively facilitated discontinuation of benzodiazepine, while maintaining good sleep quality during the tapering-off period; by the end of the tapering-off period, 14 of 18 subjects who had received melatonin, but only 4 of 16 in the placebo group, discontinued benzodiazepine (p=0.006). Sleep quality scores were significantly higher in the sustained release melatonin group (p=0.04). No serious adverse events were noted. The use of melatonin for discontinuation of drug dependencies has been described, e.g., in our European Patent No. 0724878 B1.

Suhner et al., in Aviat. Space Environ. Med. 72: 638 (2001), reported that co-administration of zolpidem 10 mg with regular release melatonin 5 mg, for jet-lag, was less effective than zolpidem alone and less well-tolerated than melatonin. The co-administered drugs caused various side-effects such as nausea, vomiting, amnesia and somnambulia to the point of incapacitation, thus suggesting that co-administration of zolpidem and melatonin would be unlikely to be of practical therapeutic use in treating conditions such as insomnia, which are related to the circadian rhythm.

However, it has unexpectedly been found in accordance with the present invention, that melatonin in a sustained-release form potentiates the effects of the non-barbiturate and non-benzodiazepine hypnotics such as zolpidem, on sedation as well as on psychomotor skills. The interaction was not additive, and it was not due to a pharmacokinetic change in blood concentrations of either zolpidem or melatonin. Most importantly, the pharmacodynamic interaction was transient and disappeared within 2 hours, while the concentrations of both drugs in blood were still high.

### SUMMARY OF THE INVENTION

The present invention thus provides in one aspect, use of melatonin in the manufacture of a medicament for short-term potentiation of the hypnotic effect in a human of at least one compound selected from the hypnotics, which are GABA-A receptor modulators zaleplon, zolpidem, zopiclone, and trazodone and wherein the medicament is adapted for sustained release of melatonin.

In another aspect, the invention provides a pharmaceutical formulation which, in addition to at least one carrier, diluent, coating or adjuvant, comprises only the following active ingredients: at least one compound selected from the hypnotics, which are GABA-A receptor modulators, zaleplon, zolpidem, zopiclone and trazodone and melatonin in sustained-release form, in an amount and form effective for short-term potentiation of the hypnotic effect of said at least one compound.

The medicament or pharmaceutical formulation is preferably further characterized by at least one of the following features:
(a) said medicament comprises at least one carrier, diluent, coating or adjuvant;
(b) said medicament is in unit dosage form;
(c) said at least one compound is present in said medicament and in an amount which, if administered in absence of melatonin, would be a sub-therapeutic amount.

### DEFINITION

The term "short-term potentiation" means potentiation for a period of not more than about 4 hours, preferably not more than about 2 hours, and particularly for a period of about one hour, +25%.

### DETAILED DESCRIPTION OF THE INVENTION

The present invention focuses on the concept of combined use of melatonin in sustained-release form and a therapeutic or sub-therapeutic dose of a non-barbiturate and non-benzodiazepine hypnotic as defined in the claims so as to effectively promote sleep initiation for patients who have difficulty falling asleep, while reducing the risk of memory impairments, psychomotor performance accidents, and subsequent tolerance and dependence,

The medicament or pharmaceutical formulation preferably includes at least one acrylic resin and is adapted for sustained release of melatonin; desirably, it is further adapted for regular release of said at least one compound.

In this connection, the sustained release properties may be achieved, e.g., by at least one of the following features, namely:
(a) by variation in the particle size of the melatonin;
(b) by use of at least two different coating materials which dissolve at different rates in the human body; and/or
(c) by varying the thickness of coating material(s) whereby the particulate melatonin is coated with different thicknesses of coating material(s) which dissolve at different rates in the human body.

The at least one hypnotic compound comprises a bicyclic fused ring system, e.g. one including at least two ring nitrogen atoms.

Exemplary such ring systems are:
the pyrazolo[1,5-a]pyrimidine skeleton, e.g. the hypnotic zaleplon;
the imidazo[1,2,-a]pyridine skeleton, e.g. the hypnotic zolpidem;
the pyrrolo[3,4-b]pyrazine skeleton, e.g. the hypnotic zopiclone; and
the triazolo[4,3-a]-pyridine skeleton, e.g. the hypnotic trazodone.

The invention will now be illustrated by the following Examples.

### EXAMPLE 1

Method. The pharmacokinetics of melatonin (2 mg sustained release formulation), zolpidem (10 mg) and their combination were assessed in 16 volunteers (12 males and 4 females). The mean age of the enrolled subjects was 59.4 years (SD 3.2), the mean Body Mass Index was 25.5 kg/m2 (SD 2.3), the mean weight was 75.8 kg (SD 11.8) and the mean height was 171.8 cm (SD 7.7). In a randomized, double-blind, crossover study, the subjects were given a tablet of placebo in the evening to establish baseline and then a tablet of melatonin, zolpidem, or a combined dose or placebo, in a random order in the evening (one night only), with a one week washout between treatments.

Blood samples were withdrawn from the subjects at pre-selected intervals after the administration of the tablets.

Results. The pharmacokinetic parameters of the two drugs when given alone and in combination are presented in Table 1:

**Table 1: Pharmacokinetic parameters of melatonin (sustained release 2 mg) and zolpidem (10 mg) when given alone and in combination**

| Drug given | Pharmacokinetic parameter | Melatonin in serum Mean (SD) | Zolpidem in serum Mean (SD) |
|---|---|---|---|
| Melatonin | Area under the curve | 5.91 (3.3) ng/ml | |
| Zolpidem | Area under the curve | - | 0.88 (0.61) mcg/ml |
| Melatonin + zolpidem | Area under the curve | 5.95 (3.9) ng/ml | 1.1 (0.7) mcg/ml |
| | | | |
| Melatonin | Time to maximum | 1.88 (1.4) h | |
| Zolpidem | Time to maximum | | 1.8 (1.2) h |
| Melatonin + zolpidem | Time to maximum | 2.13 (1.3) h | 2.0 (1.1) h |
| | | | |
| Melatonin | maximum concentration | 1.21 (0.6) ng/ml | |
| Zolpidem | maximum concentration | - | 0.22(0.11) mcg/ml |
| Melatonin + zolpidem | maximum concentration | 1.26 (0.8) ng/ml | 0.19 (0.05) mcg/ml |
| All P values of combination compared to individual drug > 0.5 (no significant differences). | | | |

Conclusions. After concomitant administration of sustained release melatonin and zolpidem, melatonin absorption is similar to the results after single dosing of sustained release melatonin. After single administration of zolpidem 10 mg, plasma concentration values of zolpidem are comparable to those after co-administration of zolpidem with sustained release melatonin. Based on the lack of pharmacokinetic interaction, there should be no differences in efficacy of zolpidem when given concomitantly with melatonin.

### EXAMPLE 2

Method. The effects of melatonin (2 mg sustained release formulation), zolpidem (10 mg), their combination and placebo, on psychomotor skills and driving performance, were assessed in 16 volunteers (12 males and 4 females). The mean age of the enrolled subjects was 59.4 years (SD 3.2), the mean Body Mass Index was 25.5 kg/m2 (SD 2.3), the mean weight was 75.8 kg (SD 11.8) and the mean height was 171.8 cm (SD 7.7).

In a randomized, double-blind, crossover study the subjects were given a tablet of placebo in the evening to establish baseline and then a tablet of melatonin, zolpidem, their combination, or placebo, in a random order in the evening with one week with no treatment in between treatments. A battery of performance tests and driving skill tests were given to the patients at pre-selected intervals after the administration of the tablet. These included psychomotor tasks for reaction test, vigilance and co-ordination: ARCI 49, Grooved Pegboard, Rivermead story, picture presentation, simple reaction time, digit vigilance task, choice reaction time, delayed picture recognition, visual tracking, driving simulator: highway driving and wake-EEG.

### Results.

Cognitive Drug research tests: No cognitive effects of sustained release melatonin dosed alone, adverse or otherwise were identified. There were several acute impairments seen with zolpidem compared to placebo, which were resolved by 12.5 hours post-dosing. The effects found with zolpidem alone, were seen across measures of attention, episodic secondary memory and motor co-ordination. When sustained release melatonin and zolpidem were co-dosed, in placebo comparisons impairments were seen for all measures at 1 hour, some of these persisting until 4 hours. At 1 hour post-dose, the impairments with co-dosing were significantly greater than those produced by zolpidem alone, and must therefore be considered synergistic interactions. At 4 hours, the impairments seen with co-dosing were similar to the effects of zolpidem alone at this time. At the 12.5 and 15 hours post-dose, there is no evidence for any effects of co-dosing the two compounds.

ARCI49: a decrease of euphoria (MBG scale) was observed 1 hour post-dosing with all groups. Four hours after administration, this effect was more pronounced with the three treatment groups as compared to placebo. An increase of dullness or slow-wittedness (LSD scale) was observed during the 4 hours post-dosing, for the three treatment groups compared to placebo. A strong significant sedative effect was noticed (increase of the PCAG scale) during the first 4 hours post-dosing in the zolpidem 10 mg and the sustained release melatonin 2 mg + zolpidem 10 mg groups, as compared to placebo. In the combined group, this effect reached the maximum at about 1 hour after administration, while in the zolpidem group, this effect increased gradually to reach the same maximal value only at about 4 hours post-dosing. Concerning the sustained release melatonin 2 mg group, a slight increase was also observed at about 4 hours post-dosing, but this effect was not significant as compared to placebo. Finally, a similar decrease of the empirical excitation (BG scale) was also noticed during the first 4 hours post-dosing in the zolpidem 10 mg and the sustained release melatonin 2 mg + zolpidem 10 mg groups, as compared to placebo. This effect corroborates the sedative effect observed. All these effects had completely passed by the next morning (at 12h30 and 15 hours post-dosing). Rivermead story: memory efficiency was decreased with zolpidem 10 mg and sustained release melatonin 2 mg + zolpidem 10 mg, for both recalls (immediate and delayed), compared to placebo and sustained release melatonin 2 mg. Immediate retrieval performance was more disturbed with zolpidem 10 mg + sustained release melatonin 2 mg, than with zolpidem 10 mg alone, while the impairment on delayed recall (amnesic effect) was equivalent in the two treatment groups. This amnesic effect observed in these two treatment groups was essentially linked to zolpidem. Indeed, sustained release melatonin 2 mg seems to potentiate the effect of zolpidem 10 mg concerning the performances in immediate memory but not for delayed memory.

Grooved Pegboard: results observed in the Grooved Pegboard test showed a slowing of the execution of the task for both hands though the fine manual coordination is not disturbed. Indeed, for both conditions (ipsi and contra lateral) a significant increase of the duration was observed at 1 and 4 hours post-dosing in the zolpidem 10 mg and sustained release melatonin 2 mg + zolpidem 10 mg groups, compared to baseline and the two other treatments (placebo and sustained release melatonin 2 mg). This increase was more pronounced in the combined treatment group, suggesting that sustained release melatonin 2 mg potentiates the effects of zolpidem 10 mg. The main slowing effect appears at 1h post-dosing and then decreases over the time.

Driving simulator: no significant difference was observed on medians of the investigated parameters (absolute speed, deviation from the speed limit and deviation from the ideal route). However, significant differences were noticed for the standard deviations of these parameters, and the number of collisions. Indeed, the standard deviations for the absolute speed and the deviations from the speed limit and ideal route parameters, were increased at 2 hours post-dosing with zolpidem 10 mg and zolpidem 10 mg + sustained release melatonin 2 mg. For absolute speed parameter, this effect was even more pronounced in the combined treatment group. These standard deviation increases suggest that driving is irregular, fluctuating not only for the speed but also for the road holding. The variations observed for the ideal route parameter, corroborate with the increased number of collisions counted at 2 hours post-dosing, in zolpidem 10 mg and sustained release melatonin 2 mg, compared with zolpidem 10 mg groups. By the next morning, this driving irregularity had disappeared, and the number of collisions was similar to the placebo and sustained release melatonin 2 mg treatment groups. At 13 hours after administration, neither drug disturbs driving abilities.

Wake EEG: In resting conditions, no major differences in alpha activity have been observed for sustained release melatonin compared to placebo. The decreases in alpha seen with zolpidem alone or zolpidem + sustained release melatonin, are in agreement with the sedative potential of zolpidem. In driving conditions, alpha activity was significantly increased under zolpidem or zolpidem + sustained release melatonin compared to sustained release melatonin alone (but not placebo). Compared to placebo, zolpidem has an increased theta rhythm on frontal leads, which is interpreted as an additional sign of sleep-inducing effects.

On Day 2, some effects revealing reduced vigilance remain present under eyes-closed conditions, which could be due to resting. Indeed, these effects were abolished under active conditions while driving or performing cognitive tests.

The most common treatment-emergent adverse event that occurred in this study was somnolence. The incidence of somnolence was similar with zolpidem and zolpidem + sustained release melatonin, but had clearly increased compared with melatonin alone and placebo. There seems to be a potentiation of central effects of zolpidem by concomitant intake of sustained release melatonin, since the intensity of adverse events was more severe with the combined treatment than with zolpidem alone; however, sustained release melatonin alone was well tolerated.

Conclusions. The effects of sustained release melatonin 2 mg treatment on performance, memory and sedation are comparable on most parameters to those observed with placebo. The present study has clearly identified a transitory pharmacodynamic interaction between sustained release melatonin and zolpidem, particularly at 1 hour following co-dosing. This had largely passed by 4 hours although the levels of the two drugs were still high in plasma, and had completely passed by 12.5 and 15 hours.

When associated with melatonin sustained release 2 mg, the impairments observed with zolpidem on mood, skill and cognitive aspects are emphasized particularly by 1 hour post-dosing. It should be noted that these interactions are potentially of clinical importance, because they should allow short-term potentiation of the effects of sub clinical doses of zolpidem, particularly during the first hour after dosing, when it is advantageous for sleep induction and also reduces the risk of further impairments by zolpidem, in view of subsequent non-potentiation.

Zolpidem treatment resulted in a significant worsening in driving skills and memory tasks in the first hours of its administration, whereas the effect of melatonin was not different from those of placebo treatment. These studies show that improvement in quality of sleep reported by patients (as is the case with zolpidem) does not necessarily indicate enhanced restorative sleep if it is not associated with improved daytime vigilance.

It should be noted that a sustained release melatonin formulation is of special interest in this respect, as it has been proven to improve sleep quality in patients with insomnia aged 55 and older, with a subsequent improvement in daytime vigilance. Melatonin is however not perceived by patients as improving sleep initiation and that aspect is well provided by zolpidem. These facts will be important for designing a new hypnotic treatment with a better safety/efficacy profile.

The present invention contemplates co-administration of melatonin in sustained-release form and the defined hypnotic, such as zolpidem. The term co-administration in this context, the purpose of which is to achieve an improved clinical outcome, may be practised by administering separate dosage forms of melatonin and hypnotic, or a combined dosage form. An illustrative Example of the preparation of a combined dosage form follows. It will be appreciated, however, that other known methods may be used for preparing a combined dosage form, such as, for example, the methods described in US 6,174,873 B1.

### EXAMPLE 3

In this Example, a two-layer tablet is prepared, which is sustained release in respect of melatonin (inner core), but regular release in respect of the exemplary hypnotic, zolpidem (outer layer). Because the outer layer undergoes immediate dissolution in the enteric system, the profile of zolpidem generated will resemble that given in Example 1, whereas because the core tablet will dissolve gradually, the profile of melatonin generated in the blood will be similar to that of Example 1 also. Method. A sustained release core melatonin tablet was first prepared by mixing together the following ingredients and compressing the mixture in a 7 mm cylindrical punch, at 2.5 tons, namely, melatonin (2 mg/tablet), and Eudragit RSPO acrylic resin carrier (Rohm Pharma), lactose and calcium hydrogen phosphate, in an approximately 2:1:2.5 ratio by weight.

An aqueous coating spray suspension is then prepared by suspending an acrylic resin (Eudragit RD 100), polysorbate 80 and talc in an approximately 10:2:5 ratio by weight, and zolpidem tartrate (5 mg/tablet) in 6 ml water per 1 g solid. The core tablet is then sprayed with the suspension to a 2 mm dried coating thickness, thus forming a coated tablet.

While this formulation should be administered in accordance with a physician's instructions, it is presently contemplated that two such tablets taken two hours before bedtime would be appropriate.

Sustained release melatonin has an effect of its own on sleep. This is demonstrated by an improvement in restorative sleep (improvement of subjective quality of sleep and subsequent improvement in daytime vigilance) as we have recently described in the patent on the use of melatonin to improve quality, and is given here as Examples 4, 5 and the delay in the cortisol peak towards the morning hours that is seen with the sustained release but not with the regular release formulation (Example 6). This effect may be responsible for the enhancement of restorative sleep.

### EXAMPLE 4

Method. The effect of a sustained release formulation of melatonin on sleep quantity and quality in 40 elderly primary insomnia patients (aged 76 years) (SD 8), were studied in a randomized, double-blind, two parallel group study. The subjects were treated for 3 weeks every evening with melatonin (2 mg sustained release formulation) or placebo. Full-night polysomnographic recordings were performed on the last two days of treatment to measure quantitative aspects of sleep. On each morning following sleep recording in the laboratory, a battery of psychomotor tests was taken by all patients to assess daytime vigilance. In addition, patients recorded every day in diaries their perceived quality of sleep the previous night.

Results. The results show beneficial effects of melatonin on sleep initiation, similar to the effects of hypnotic drugs. In contrast to this apparently hypnotic effect, psychomotor skills were significantly higher in the melatonin group compared to the placebo-treated group: Significant treatment effects for the Critical Flicker fusion test and Total Reaction Time under melatonin vs. placebo were observed at the end of treatment.

Conclusions. These results thus show for the first time the association of hypnotic effect (shortening of sleep latency) by melatonin with enhanced daytime vigilance in primary insomnia patients, suggesting that the restorative value of sleep has increased in these patients. When using hypnotic drugs, shortening of sleep latency and improved quality of sleep is associated with impaired psychomotor skills in the morning, or at best no significant deterioration. No hypnotic drug has ever been shown to increase daytime vigilance. Surprisingly, in their diaries, patients did not evaluate the ease of getting to sleep as being better with melatonin compared to placebo. In fact, the patients judged their quality of sleep to be improved with melatonin but not placebo treatment. The restorative value of sleep may thus be associated with a perceived improvement in quality of sleep.

### EXAMPLE 5

Method. The effect of a sustained release formulation of melatonin on subjectively assessed sleep quality and daytime vigilance in 170 elderly primary insomnia patients (aged 68.5 years) (SD 8.3) was studied in a randomized, double-blind, two parallel group study. The subjects were treated for 2 weeks with placebo to establish baseline characteristics and then for 3 weeks with melatonin (2 mg per night of sustained release formulation) or placebo. On the last three days of the baseline and treatment periods patients were asked to assess the quality of their sleep the previous night and their feeling in the morning. The quality of sleep question was "How would you compare the quality of sleep using the medication with non-medicated (your usual) sleep?" The patients marked the level of their perceived quality of sleep on a 100 mm, non-hatched horizontal line with two endpoints. The left endpoint labeled "more restless than usual" and the right endpoint is labeled "more restful than usual". The waking state question was "How do you feel now?" The patients marked the level of their perceived waking state on a 100 mm, non-hatched horizontal line with two endpoints. The left endpoint labeled "tired" and the right endpoint is labeled "alert". The distance of the patient mark from the right endpoint in mm was measured. (a reduction in value therefore indicates a better sleep or less tired state). The mean distance across the three nights was calculated.

Results. It was found that both quality of sleep and daytime alertness significantly improved with sustained release melatonin compared to placebo (Table 2) showing a link between improved restful sleep and less fatigue in the morning.

**Table 2: Effects of sustained release melatonin and placebo on subjectively assessed quality of sleep and daytime alertness in primary insomnia patients.**

| Response | Melatonin, change in mm mean (SE) | Placebo, change in mm mean (SE) |
|---|---|---|
| Change in perceived quality of sleep | -24.3 (2.6)* | -17.6 (2.1) |
| Change in perceived daytime alertness | -16.8 (2.7)* | -6.6 (2.0) |

| | | |
|---|---|---|
| *The difference from placebo is significant (p<0.05) | | |

Conclusions. These results show that melatonin enhanced the restorative value of sleep in these primary insomnia patients.

### EXAMPLE 6

Method. The following experiments were performed in a double-blind, placebo controlled crossover fashion. Each patient received all three kinds of tablets (placebo, regular release and sustained release), but in random order not known to the patient or the staff.

Results. Administration of melatonin (2 mg) in a sustained release formulation (SR-Mf), once daily at 10 PM, for one week, to eight healthy elderly persons suffering from insomnia, resulted in a significant increase in their sleep efficiency but not sleep latency. (Sleep efficiency is the amount of time spent asleep from total time in bed; sleep latency is the time taken to fall asleep from first lights-off). On the other hand, treatment of the same individuals with melatonin (2 mg) in a regular release formulation .(RM) did not improve sleep efficiency but shortened sleep latency compared to placebo treatment of the same subjects. These results can be explained by the short half-life of melatonin in the blood. Namely, the sustained release formulation produces lower blood levels of the hormone for extended periods of time and thus its effects may start slowly but are significant later on during the night.

The cortisol level in these patients was assessed by urinary excretion of the hormone at 2 hour intervals over a 24 hour period. In the placebo treatment group, patients displayed a cortisol rhythm which reached its peak at 8:36 AM and the cortisol then declined, as is known for subjects above 40 years of age. The mean 24 hour excretion rate/hour (which approximated blood concentrations) of the cortisol in urine in the control group was 3.2 microgram/hour. The amplitude of the rhythm (i.e. maximal deviation of the mean 24 h to maximum or minimum excretion rate) was 1.8 mg/hour.

After treatment for 1 week with the regular release melatonin the overall amount of cortisol excreted was reduced. The .mean 24 hour excretion rate decreased to 2.5 mg/hour and the amplitude decreased to 1.0 mg/hour. In addition there was a slight backwards shift in the time of the peak, which occurred at 8:27 AM. Anticipation of the cortisol rhythm after administration of regular release melatonin is compatible with observations made by Terzolo et al., J. Pineal Research, 1990, 9: 113-124. However, a decrease in mean 24 hour levels and amplitude of the cortisol rhythm was not observed by Terzolo.

After one week's treatment with sustained release melatonin, it was found that like the regular melatonin, secretion of cortisol was attenuated (mean 24 h rate was 2.5 mg/hour) and the amplitude 1.2 mg/hour (as with the regular release), but the peak was delayed significantly to later in the day and occurred at 12:06 PM. Thus, the peak was delayed by administration of sustained release melatonin instead of being the same or slightly advanced. The same cortisol profile was also found in these patients after 1 month's treatment with the sustained release formulation (mean 24 hour excretion 2.5 mg/hour, amplitude 1.0 mg/hour and peak time 12:08 hours). Conclusions. These results show that the response of the body to melatonin is not obvious: the body reads the melatonin profile and not just the fact that it is present at some time. Interestingly, in humans younger than 40 years, it is known that the cortisol rhythm is also delayed compared to older individuals. Hence, the cortisol profile generated in the elderly after the sustained release melatonin treatment is similar to that in younger individuals.

Discussion. An inverse relationship has been documented in humans between cortisol and quality of sleep, i.e. as sleep quality and quantity decline, levels of the adrenal hormone cortisol increase. It may be noted that cortisol is a stress hormone, and its high levels at night may prevent restorative sleep. The present experiment shows that administration of regular release melatonin can lower cortisol production, but that administration of sustained release melatonin both lowers the cortisol level and delays its peak and thus can improve sleep during the dawn hours.

With hypnotic drugs as defined for the purpose of the present invention, such as zolpidem, it is crucial that elimination will be rapid and that no drug will remain in the morning. Because the drug only affects the initiation of sleep, it is useful to augment its effects in the first hour so as to get maximal efficacy with a lower dose and avoid its detrimental effects later on in the night. The intrinsic effects of melatonin, when co-administered with e.g. zolpidem, are maintained. The combination of melatonin and zolpidem according to the invention will thus allow improvement of subjective sleep latency (that is not perceived with melatonin alone) while avoiding the bad effects of zolpidem later on in the night (on memory and coordination).

### EXAMPLE 7

Method. The effect of a sustained release formulation of melatonin on subjectively assessed sleep quality and daytime vigilance in 5 primary insomnia patients aged 55 years and older, who were already taking 10 mg zolpidem per night, were studied. The subjects were treated for 2 weeks with placebo to establish baseline characteristics and then for 3 weeks with melatonin (2 mg per night of sustained release formulation). On the last three days of the baseline and treatment periods patients were asked to assess the quality of their sleep the previous night. The quality of sleep question was "How would you compare the quality of sleep using the medication with non-medicated (your usual) sleep)?" The patients marked the level of their perceived quality of sleep on a 100 mm, non-hatched horizontal line with two endpoints. The left endpoint is labeled "more restless than usual" and the right endpoint is labeled "more restful than usual". The distance of the patient mark from the right endpoint in mm was measured. (a reduction in value therefore indicates a better sleep or less tired state). The mean distance across the three nights was calculated. Response was defined as a mean improvement in the 3 nights of 10 mm on the 100 mm visual analog scales.

Results. It was found that 3 of the 5 patients that were taking zolpidem responded to the concomitant therapy with melatonin (60%). This value is equivalent to that obtained in parallel studies with patients who had not been taking zolpidem concomitantly.

Conclusions. The improvement of quality of sleep upon concomitant therapy with melatonin can be ascribed to melatonin and not zolpidem, since patients were taking zolpidem already at baseline. In addition, the synergy between the two drugs is particularly evident in the first hour after administration and should not affect the all night sleep quality. Moreover, these data show that the clinical efficacy of melatonin (after the synergy period) is maintained when given concomitantly with zolpidem. Discussion. Since it is known that zolpidem does not alter the profile of endogenous melatonin, and that melatonin does not bind to the benzodiazepine receptor, it is clear that the potentiation (or synergy) in accordance with the present invention, is due neither to replacement of melatonin deficiency by zolpidem, nor to binding of both agents to the same receptor.

Particular embodiments of the invention have been particularly described hereinabove.

## Claims

1. Use of melatonin in the manufacture of a medicament for short-term potentiation of the hypnotic effect in a human of at least one compound selected from the hypnotics, which are GABA-A receptor modulators zaleplon, zolpidern, zopidone, and trazadone, and wherein the medicament is adapted for a sustained release of melatonin.

2. Use according to claim 1, which is further **characterized by** at least one of the following features:
(a) said medicament comprises at least one carrier, diluent, coating or adjuvant;
(b) said medicament is in unit dosage form;
(c) said at least one compound is present in said medicament and in an amount which, if administered in absence of melatonin, would be a sub-therapeutic amount.

3. Use according to claim 2, wherein said medicament includes at least one acrylic resin.

4. A pharmaceutical formulation which, in addition to at least one carrier, diluent, coating or adjuvant, comprises only the following active ingredients:
at least one compound selected from the hypnotics, which are GABA-A receptor modulators, zaleplon, zolpidern, zopiclone, and trazodone and melatonin in sustained-release form, in an amount and form effective for short-term potentiation of the hypnotic effect of said at least one compound.

5. A pharmaceutical formulation according to claim 4, which is further **characterized by** at least one of the following features:
(a) said formulation is in unit dosage form:
(b) said at least one compound is present in said formulation in an amount which, if administered in absence of melatonin, would be a sub-therapeutic amount.

6. A pharmaceutical formulation according to claim 4, which includes at least one acrylic resin.

7. A pharmaceutical formulation according to claim 4, which is further adapted for regular release of said at least one compound.

## Patentansprüche

1. Verwendung von Melatonin in der Herstellung eines Medikaments für kurzfristige Potenzienng der hypnotischen Wirkung bei einem Menschen von mindestens einer Verbindung, ausgewählt aus den Hypnotika, welche die GABA-A-Rezeptor-Modulatoren Zaleplon, Zolpidern, Zopiclon und Trazodon sind, und wobei das Medikament für eine verzögerte Freisetzung von Melatonin angepaßt ist.

2. Verwendung gemäß Anspruch 1, welche weiter durch mindestens eines der folgenden Merkmale **gekennzeichnet** ist:
(a) das Medikament umfaßt mindestens einen Träger, ein Verdünnungsmittel, eine Beschichtung oder einen Hilfsstoff;
(b) das Medikament ist in Einheitsdosierungsforrn;
(c) die mindestens eine Verbindung ist in dem Medikament vorhanden und in einer Menge, welche, wenn in Abwesenheit von Melatonin verabreicht, eine subtherapeutische Menge sein würde.

3. Verwendung gemäß Anspruch 2, wobei das Medikament mindestens ein Acrylharz einschließt.

4. Pharmazeutische Formulierung, welche, zusätzlich zu mindestens einem Träger, einem Verdünnungsmittel, einer Beschichtung oder einem Hilfsstoff, nur die folgenden Wirkstoffe umfaßt:
mindestens eine Verbindung, ausgewählt aus den Hypnotika, welche die GABA-A-Rezeptor-Modulatoren Zaleplon, Zolpidem, Zopiclon und Trazodon sind, und Melatonin in einer Form mit verzögerter Freisetzung in einer Menge und Form, wirksam tür kurzfristige Potenzierung der hypnotischen Wirkung der mindestens einen Verbindung.

5. Pharmazeutische Formulierung gemäß Anspruch 4, welche weiter durch mindestens eines der folgenden Merkmale gekenntzeichnet ist:
(a) die Formulierung ist in Einheitsdosierungsform ;
(b) die mindestens eine Verbindung ist in der Formulierung in einer Menge vorhanden, welche, wenn in Abwesenheit von Melatonin verabreicht, eine subtherapeutische Menge sein würde.

6. Pharmazeutische Formulierung gemäß Anspruch 4, welche mindestens ein Acrylharz einschließt,

7. Pharmazeutische Formulierung gemäß Anspruch 4, welche weiter für reguläre Freisetzung der mindestens einen Verbindung angepaßt ist.

## Revendications

1. Utilisation de mélatonine dans la fabrication d'un médicament destiné à la potentialisation à court terme de l'effet hypnotique chez un être humain d'au moins un composé choisi parmi les hypnotiques, qui sont des modulateurs du récepteur GABA-A, zaleplon, zolpidem, zopiclone, et trazodone, le médicament étant conçu pour une libération prolongée de la mélatonine.

2. Utilisation selon la revendication 1, qui est en outre **caractérisée par** au moins une des caractéristiques suivantes:
(a) ledit médicament contient au moins un véhicule, diluant, enrobage, ou adjuvant;
(b) ledit médicament se présente sous une forme posologique unitaire;
(c) ledit au moins un composé est présent dans ledit médicament et en une quantité qui, si on l'administrait en l'absence de mélatonine, serait une quantité infrathérapeutique.

3. Utilisation selon la revendication 2, dans laquelle ledit médicament contient au moins une résine acrylique.

4. Formulation pharmaceutique qui, en plus de contenir au moins un véhicule, diluant, enrobage ou adjuvant, contient seulement les principes actifs suivants:
au moins un composé choisi parmi les hypnotiques, qui sont des modulateurs du récepteur GABA-A, zaleplon, zolpidem, zopiclone, et trazodone, et de la mélatonine sous une forme à libération prolongée, en une quantité et sous une forme efficace pour la potentialisation à court terme de l'effet hypnotique dudit au moins un composé.

5. Formulation pharmaceutique selon la revendication 4, qui est en outre **caractérisée par** au moins une des caractéristiques suivantes:
(a) ladite formulation se présente sous une forme posologique unitaire;
(b) ledit au moins un composé est présent dans ladite formulation en une quantité qui, si on l'administrait en l'absence de mélatonine, serait une quantité infrathérapeutique.

6. Formulation pharmaceutique selon la revendication 4, qui contient au moins une résine acrylique.

7. Formulation pharmaceutique selon la revendication 4, qui est en outre conçue pour la libération régulière dudit au moins un composé.
